(19) Europäisches Patentamt
European Patent Office
Office européen des brevets

(11) EP 0 648 744 B1

(12) EUROPEAN PATENT SPECIFICATION

(45) Date of publication and mention of the grant of the patent: **21.01.1998 Bulletin 1998/04**

(51) Int Cl.[6]: **C07D 211/22**, A61K 31/445

(21) Application number: **94112867.0**

(22) Date of filing: **18.08.1994**

# (54) Phenylalkanolamine derivatives as antagonists of the NMDA receptor

Phenylalkanolamin Derivate mit NMDA Rezeptor antagonistischer Wirkung

Dérivés de phénylalkanolamine avec une activité sur le réceteur NMDA

(84) Designated Contracting States:
**AT BE CH DE DK ES FR GB GR IE IT LI LU MC NL PT SE**

(30) Priority: **02.09.1993 US 116385**

(43) Date of publication of application:
**19.04.1995 Bulletin 1995/16**

(73) Proprietor: **F. HOFFMANN-LA ROCHE AG
4002 Basel (CH)**

(72) Inventors:
- **Mohacsi, Erno**
  **Summit, New Jersey 07901 (US)**
- **O'Brien, Jay Philip**
  **Cedar Grove, New Jersey 07009 (US)**

(74) Representative: **Poppe, Regina et al
F.Hoffmann-La Roche AG
Patent Department(PLP),
124 Grenzacherstrasse
4070 Basel (CH)**

(56) References cited:
**EP-A- 0 163 537**
**EP-A- 0 174 565**
**EP-A- 0 398 578**
**WO-A-91/05787**
**WO-A-92/06957**
**WO-A-92/08724**
**DE-A- 2 052 537**

- **J.MED.CHEM., vol.34, 1991, WASHINGTON pages 3085 - 3090 CHENARD,B.L. ET AL. 'Separation of alpha-1 adrenergic and NMDA antagonist Activity in a Series of Ifenprodil Compounds'**
- **J.MED.CHEM., vol.34, 1991, WASHINGTON pages 90 - 97 ORNSTEIN,P.L. ET AL. '4-(Tetrazolylalkyl)p iperidine-2-carboxylic acids. Poetent and selective....'**
- **DRUGS, vol.44, no.3, 1992 pages 279 - 292 ROGAWSKI,M.A. 'The NMDA Receptor, NMDA Antagonists and Epilepsy Therapy'**
- **J.MED.CHEM., vol.33, 1990, WASHINGTON pages 2916 - 2924 HAYS,S.J. ET AL. 'New and versatile Approaches to the Synthesis of CPP-related competitive NMDA antagonists...'**
- **J.ORG.CHEM.53,5415(1988)**

Remarks:
The file contains technical information submitted after the application was filed and not included in this specification

EP 0 648 744 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The invention relates to a compound of the formula

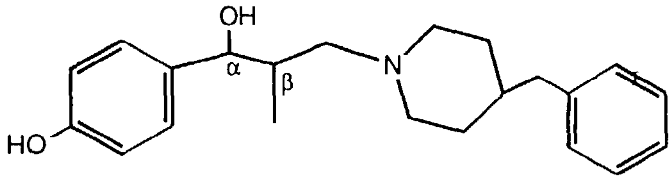

I

enantiomers or diastereomers thereof and to pharmaceutically acceptable salts thereof.

The compound of formula I contains two asymmetric centers at the α-and β-positions. Accordingly, the compound of formula I can be in form of two racemates, enantiomers or diastereomers, that is, erythro (R*,S*) or threo (R*,R*) isomers.

As used herein, the terms "erythro" and "threo" refer to the relative configurations of the hydroxy and the methyl substituent, at the α- and β-positions of the compounds of formula I' and I" as shown below, which are racemates. However, the enantiomers of the erythro and threo racemates of formula I' and I" are part of the invention.

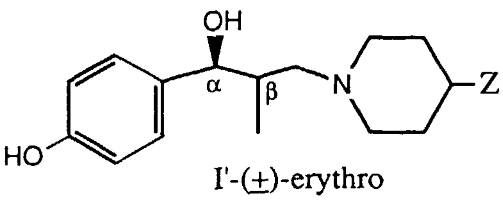

I'-(±)-erythro

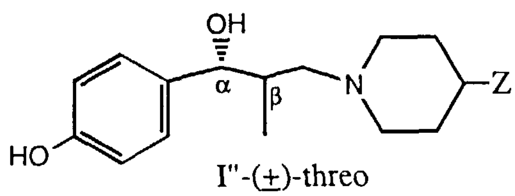

I"-(±)-threo

Z is $-CH_2C_6H_5$

The compound of formula I reduces adverse effects of neurotoxic injury and thus is useful in the treatment of neurodegenerative diseases, such as, stroke, ischemia, hypoxia, hypo-glycemia, epilepsy, and the like.

The compound of the present invention is new. In J. Med. Chem. 34, 3085, (1991) are described ifenprodil and analog compounds, which differ from the compound of the present invention by the length of the linking group between the phenyl ring and the piperidine group.

Objects of the present invention are the compound of formula I and its pharmaceutically acceptable salts per se and for use as therapeutically active substances, the manufacture of this compound, a medicament containing it and the manufacture of such a medicament, as well as the use of the compound of formula I and its pharmaceutically acceptable salts in the control or prevention of illnesses or in the improvement of health, especially in the control or prevention of neurodegenerative diseases, such as stroke, ischemia, hypoxia, hypoglycemia, epilepsy, and the like.

Exemplary compounds enclosed in the present invention are:

(R*,S*)-rac.-α-(4-hydroxyphenyl)-β-methyl-4-(phenylmethyl)-1-piperidinepropanol;

[R-(R*,S*)]-α-(4-hydroxyphenyl)-β-methyl-4-(phenylmethyl)-1-piperidinepropanol;

[S-(R*,S*)]-α-(4-hydroxyphenyl)-β-methyl-4-(phenylmethyl)-1-piperidinepropanol; and

(R*,R*)-rac-α-(4-hydroxyphenyl)-β-methyl-4-(phenylmethyl)-1-piperidinepropanol.

In accordance with the present invention the compound of formula I and its pharmaceutically acceptable salts can be prepared by a process which comprises

a) reducing a compound of the formula

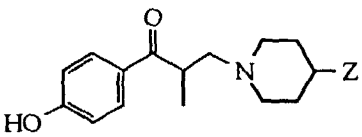

II

wherein Z is $-CH_2C_6H_5$,

with hydrogen in the presence of a suitable catalyst such as palladium on carbon, or with a complex alkali metal hydride, such as lithium aluminium or potassium borohydride, or

b) debenzylating a compound of the formula

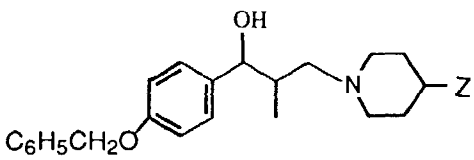

III

wherein Z is benzyl, or

c) for the manufacture of an optically pure compound of formula I, resolving a racemic mixture into its enantiomeric components, or

d) if desired, converting a compound of formula I into a pharmaceutically acceptable salt.

The reaction conditions for the above process variants a)-d) are described in more details hereinafter in Reaction Schemes 1-3

## SCHEME 1

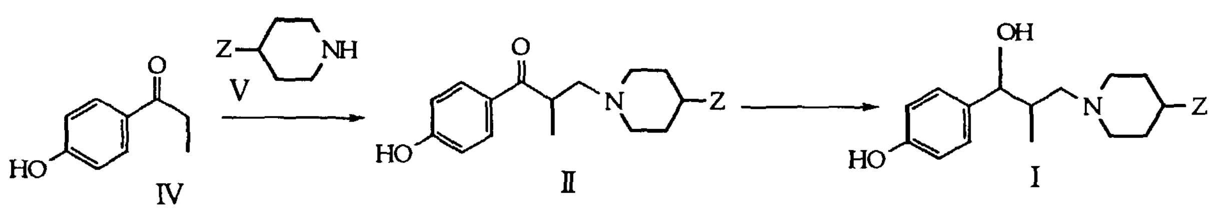

IV V II I

wherein Z is benzyl.

As set forth in Scheme 1, a compound of formula IV, a known compound, is reacted with a compound of formula V, a known compound, or which can be prepared by known methods, and paraformaldehyde to form a corresponding compound of formula II. In carrying out this reaction, temperature and pressure are not critical. Generally, it is preferred to utilize temperatures from room temperature to 100°. The reaction is usually carried out in water, ethanol or dimethylformamide, preferably dimethylformamide.

A compound of formula II is reduced by catalytic hydrogenation in the presence of palladium on carbon at room temperature in, preferably, ethanol and at 50 p.s.i. The racemic erythro isomer of formula I can be isolated from the mixture by chromatography.

Scheme 2

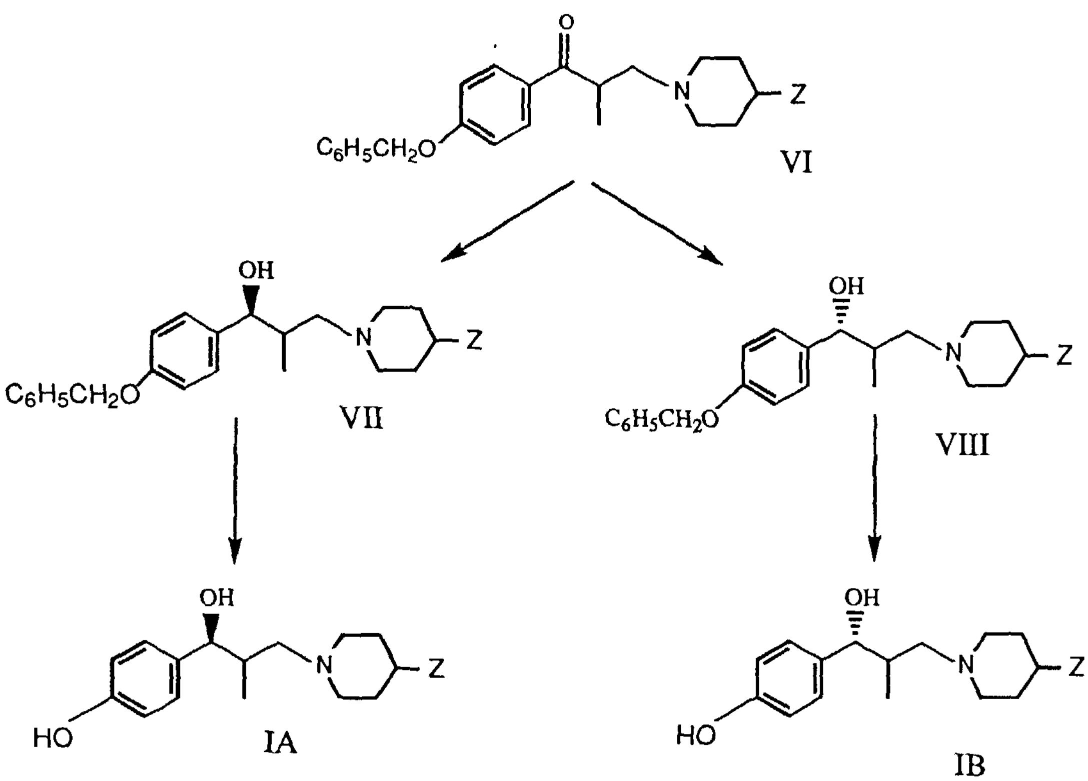

VI, VII, VIII, IA, IB

wherein Z is as described above.

A compound of formula VI forms a mixture of racemic isomers, compounds of formulas VII and VIII when reduced in the presence of a complex alkali metal hydride, such as, lithium aluminum hydride in tetrahydrofuran. Such a mixture can be separated by chromatography. The compounds of formulas VII and VIII are debenzylated separately to the racemic erythro (R*,S*) and threo (R*,R*) diastereomers, compounds of formulas IA and IB, respectively. The reaction is carried out by catalytic transfer hydrogenation using 10% palladium on carbon and ammonium formate in acetone at reflux temperature.

## SCHEME 3

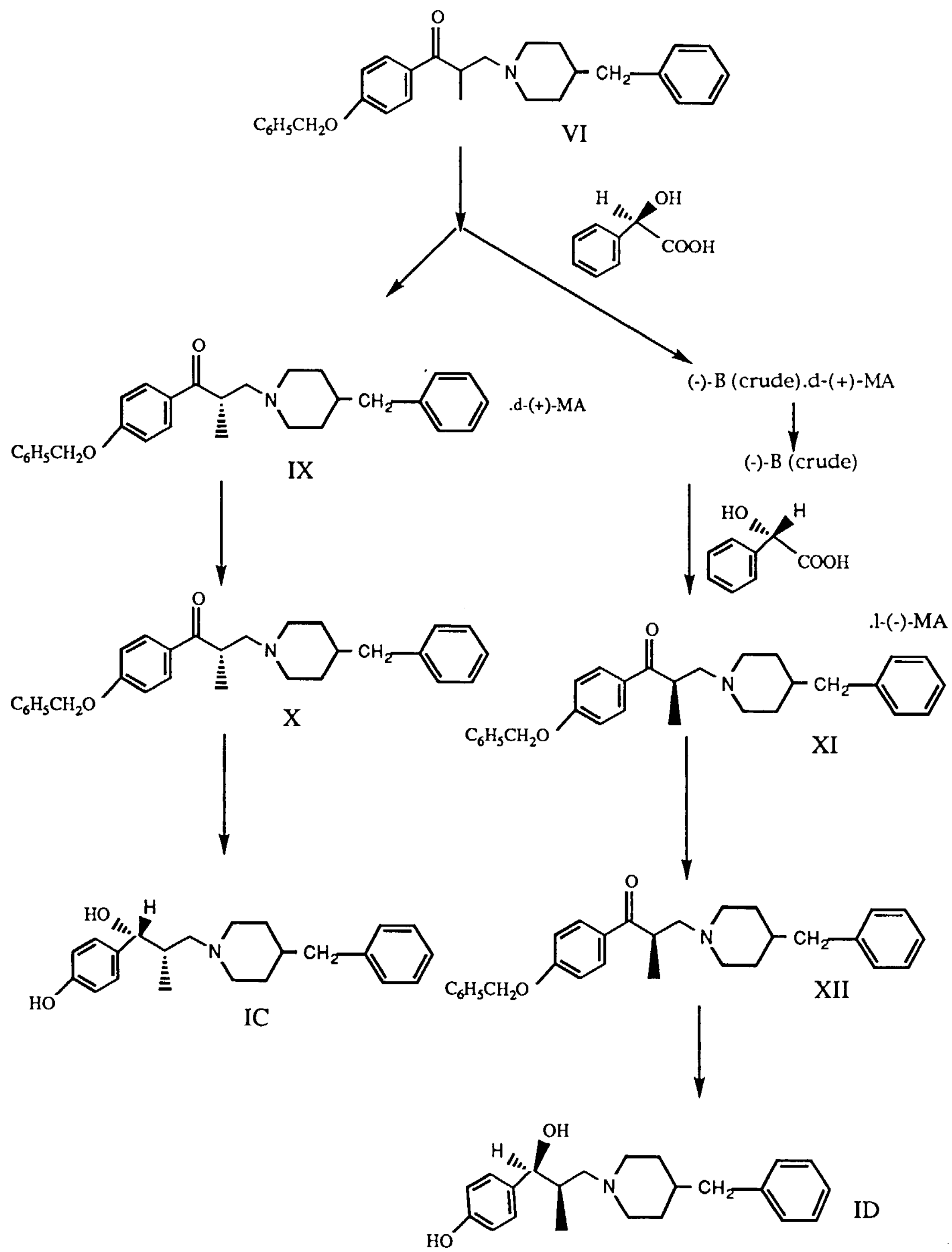


VI IX X XI IC XII ID

As set forth in Scheme 3, the compound of formula VI in acetone is treated with a resolving agent such as d-(+)-mandelic acid, which is also called (S)-α-hydroxybenzoic acid, and the resulting solution is allowed to crystallize at about room temperature. The crystals are a salt of formula IX comprising the resolving agent and the (+)-enantiomer of the compound of formula VI. The soluble salt is that of the (-)-enantiomer of the compound of formula VI in solution with the resolving agent.

The above solution is concentrated and the residue (-)-B(crude) d-(+)-MA, wherein MA is mandelic acid and B is a base, is treated in water with a base, such as, sodium hydroxide or more preferably with concentrated ammonium

hydroxide and the resulting suspension is extracted with an organic solvent, such as, methylene chloride and concentrated to give the crude (-)-enantiomer. The crude (-)-enantiomer can be further purified by dissolving in hot acetone and treating the resulting solution with 1-(-)-mandelic acid, also known as (R)-α-hydroxybenzoic acid. The crystals are a salt of formula XI of the resolving agent and the (-)enantiomer of the compound of formula VI. The salt can be suspended in water and treated with a base, such as, concentrated ammonium hydroxide, and the resulting suspension can be extracted with an organic solvent, such as, methylene chloride. The (-)-enantiomer of formula XII can be isolated from the solution by evaporation of the solvent and can be used for reduction to the compound of formula ID.

The salt of formula IX can be treated in water with a base, such as, sodium hydroxide or more preferably with concentrated ammonium hydroxide, then the aqueous suspension is extracted with an organic solvent, such as, methylene chloride and concentrated to obtain the (+)-enantiomer of formula X, which can be used for reduction to the compound of formula IC.

Compounds of formulas X and XI are reduced to (-)-erythro and (+)-erythro compounds of formulas IC and ID, respectively, by hydrogenation in the presence of palladium on carbon in acetic acid at room temperature and 50 p.s.i.

The compound of formula I is active as non-competitive NMDA receptor antagonists and is therefore, useful as neuroprotecting agents, for example, in the treatment of injury to central neurons associated with ischemia, hypoxia, hypoglycemia, epilepsy, Huntington's disease or Alzheimer's disease.

The activity of the compound of formula I can be demonstrated by the following:

1. $^{3}$H-MK 801 (dizocilpine) binding in vitro

Method

$^{3}$H-MK 801 binding was performed as described in Ransom, R.W. and Stec, N.L., J. Neurochem. 51 (1988) 830-836, with extensively washed rat brain membranes prepared according to the following scheme. Frozen whole rat brain was thawed and cerebellum and medulla oblongata were removed on ice. The tissue was then homogenized with an Ultra Turrax (maximal energy) during 30 sec at 4°C in 50 volumes of Tris•HC1 (5mM) with ethylenediaminetetraacetic acid (EDTA) disodium (10 mM, pH = 7.4). The homogenate was centrifuged at 48'000 x g for 10 min. The pellet was rehomogenized with the same volume of the same buffer and the homogenate was then incubated at 37°C for 10 min. After centrifugation the pellet was rehomogenized under the same conditions and frozen at -80°C in 35 ml fractions for at least 16 hours and not more than 2 weeks.

On the day of experiment, the homogenate was thawed 15 minutes at 37°C and centrifuged as above. The pellet was rehomo-genized in 25 volumes of Tris•HC1 buffer (5mM, pH = 7.4) and the homogenate was recentrifuged. This procedure was repeated twice. The final pellet was rehomogenized in 25 volumes of Tris•HC1 buffer (5 mM, pH = 7.4) related to the original wet weight and used as such in the assay. Final concentration in the assay was 20 mg/ml.

300 µl of tracer solution (final concentration 5 nM $^{3}$H-MK 801) in Tris•HC1 buffer (5 mM, pH 7.4) were incubated with 100 µl of test solution (100 µl of Tris•HC1 buffer for the total binding or 100 µl of buffer plus N-(1-(2-thienyl) cyclohexyl)piperidine (TCP) (final concentration 0.1 µM) for non specific binding). Glutamate, glycine and spermidine (final concentrations 1nM) were added together in 100 µl volume. 500 µl of homogenate (final concentration 20 mg/ml) were then added in the plastic reaction tubes. After 2 hours of incubation at room temperature, the suspension was filtered (Whatmann GF/B, presoaked in 0.05% polyethylenimine for 2 hours) and washed 5 times with 3 ml of cold buffer. The air-dried (3 min) filters were counted in 10 ml of Ultima-gold (Packard) after agitation (10 min.) and standing for 2 hours at 3°C in vials.

The results are set forth in Table I.

TABLE I

| Compound | $IC_{50}$ µM |
|---|---|
| Ex. 8 | 0.005 |
| Ex. 9 | 0.006 |
| Ex. 15 | 0.0013 |
| Ex. 16 | 0.003 |
| ifenprodil | 0,052 |

These are high affinity values determined from biphasic displacement curves.

2. Acute Glutamate Neurotoxicity

Method

Single cell suspensions were prepared from embryonic rat cortices (E16-17) by digestion with dispase 2.4 U/ml (BOEHRINGER) and subsequent trituration with fire polished pasteur pipettes. The cells were then plated on poly-D-lysine (SIGMA) coated 96 well microtiter plates (NUNC, $10^5$ cells/well) in a total volume of 100 µl Dulbeccos modified essential medium (DMEM, GIBCO) supplemented with 10% horse serum and penicillin/streptomycin (SIGMA). 5 days later, non-neuronal cell division was halted by exposure to $10^{-5}$ cytosine arabinoside combined with a 50% exchange of culture medium. The cultures were used for neurotoxicity assays from 8-12 days in vitro.

Acute glutamate toxicity was performed according to D.W. Choi. J. Koh and S. Peters, J. Neurosci. 8 (1988) 185-196, in 100 µl of a control salt solution (CSS: 120 mM NaCl, 5.4 mM KCl, 0.8 mm $MgCl_2$, 1.8 mM $CaCl_2$, 25 mM Tris HCl (pH 7.4 at 25°C) and 15 mM glycose) with 500 µM glutamate for 5 to 30 minutes at room temperature with or without addition of substances to be tested.

After washing, the cultures were maintained in 100 µl CSS overnight at 37°C. For quantitation of neurodegeneration, lactate dehydrogenase was measured in the cell culture supernatant as described by J.G. Klingman, D.M. Hartley and D.W. Choi, J. Neurosci. Meth. 31 (1990) 47-51 using a BIOMEK workstation (BECKMAN). Percentage of neuronal degeneration was calculated taking the difference of unprotected and maximally protected cultures (with a reference NMDA-receptor antagonist) as 100%. From dose response curves $IC_{50}$ values were calculated.

The results are set forth in Table II.

TABLE II

| Compound | $IC_{50}$ µM |
|---|---|
| Ex. 8 | 0.04 |
| Ex. 9 | 0.11 |
| Ex. 15 | 0.06 |
| Ex. 16 | 0.08 |
| ifenprodil | 0,40 |

The compound of formula I above forms pharmaceutically acceptable acid addition salts. Thus, the compound of the present invention form pharmaceutically acceptable acid addition salts including, but not limited to HCl, HBr, $HNO_3$, $H_2SO_4$, $H_3PO_4$, $CH_3SO_3H$,
$CH_3C_6H_4SO_3H$, $CH_3CO_2H$, $C_6H_5COOH$, gulonic, tartaric, mandelic and succinic.

The compound of formula I and it salts, as herein described, can be incorporated into standard pharmaceutical dosage forms, for example, they are useful for oral or parenteral application with the usual pharmaceutical adjuvant material, for example, organic or inorganic inert carrier materials, such as, water, gelatin, lactose, starch, magnesium stearate, talc, vegetable oils, gums, polyalkyleneglycols and the like. The pharmaceutical preparations can be employed in a solid form, for example, as tablets, suppositories, capsules, or in liquid form, for example, as solutions, suspensions or emulsions. Pharmaceutical adjuvant materials can be added and include preservatives, stabilizers, wetting or emulsifying agents, salts to change the osmotic pressure or to act as buffers. The pharmaceutical preparations can also contain other therapeutically active substances.

The daily dose of the compound of formula I to be administered varies with the particular compound employed, the chosen route of administration and the recipient. Representative of a method for administering the compounds of formula I is by the oral and parenteral type administration route. An oral formulation of a compound of formula I is preferably administered to an adult at a dose in the range of from 150 mg to 1.5 gm per day. A parenteral formulation of the compound of formula I is preferably administered to an adult at a dose in the range of from 5 to 500 mg per day.

The invention is further illustrated in the following examples, whereby compounds of examples 8, 9, 15 and 16 describe the preparation of compounds, which fall within the scope of claim 1.

EXAMPLE 1

rac.-3-Methyl-4-oxo-4-[4-(phenylmethoxy)phenyl]butanoic acid methyl ester

To a solution of 5.5 ml (0.055 mol) of potassium hexamethyl-disilazide (20 wt % in THF) in 20 ml of tetrahydrofuran chilled to -78°C was added dropwise a solution of 12.0 g (0.05 mol) of 4-benzyloxy-propiophenone in 30 ml of tetrahydrofuran over a period of 20 minutes. After stirring at -78°C for 1 hr, a solution of 9.0 g (0.06 mol) of methyl bromoacetate

in 25 ml of tetrahydrofuran (dry) was added dropwise to the reaction mixture over a period of 30 minutes at -78°C then stirred at this temperature for 1 hr and allowed to warm to room temperature. It was then poured onto 1N hydrochloric acid and extracted with ethyl acetate. The combined organic solutions were washed with brine, dried ($MgSO_4$) and the solvent was removed under reduced pressure to give 19.2 g of crude racemic-3-methyl-4-oxo-4-[4-(phenylmethoxy) phenyl] butanoic acid methyl ester, which was chromatographed on silica gel (300 g). Elution of the column with methylene chloride-ethyl acetate (8:2; v/v), fractions 12-35 after removal of the solvent afforded 7.1 g (42%) of racemic-3-methyl-4-oxo-4-[4-(phenylmethoxy)phenyl] butanoic acid methyl ester b.p. 205-207°C (0.2 mmHg).

EXAMPLE 2

rac.-3-Methyl-4-oxo-4-[4-(phenylmethoxy)phenyl]butanoic acid

A mixture of 11.2 g (0.0046 mol) of racemic-3-methyl-4-oxo-4-[4-(phenylmethoxy)phenyl]butanoic acid methyl ester, 120 ml of tetrahydrofuran, 12 ml of water and 60 ml of 2N sodium hydroxide was stirred at room temperature for 3 hrs. The reaction mixture was diluted with water (30 ml) and extracted with methylene chloride. The organic phase was dried ($MgSO_4$) and the solvent was removed under reduced pressure to give 11.1 g of crude racemic-3-methyl-4-oxo-4-[4-(phenylmethoxy)phenyl]butanoic acid, which was crystallized from ethyl acetate to provide 9.6 g (69%) of racemic-3-methyl-4-oxo-4-[4-(phenylmethoxy)phenyl]butanoic acid, m.p. 147-148°C.

EXAMPLE 3

rac.-2-Methyl-1-[4-(phenylmethoxy)phenyl]-4-[4-(phenylmethyl)-1-piperidinyl]-1,4-butanedione

A mixture of 8.0 g (0.0027 mol) of racemic-3-methyl-4-oxo-4-[4-(phenylmethoxy)phenyl]butanoic acid, 4.8 g (0.027 mol) of N-benzylpiperidine, 6.4 g (0.031 mol) of 1,3-dicyclohexylcarbodiimide and 4.0 g (0.029 mol) of 1-hydroxybenzotriazole in 200 ml of dry dimethyl-formamide was stirred at room temperature for 48 hrs. The reaction mixture was poured onto 1N hydrochloric acid and the aqueous suspension was extracted with ethyl acetate. The organic extracts were washed with 2N sodium hydroxide, then brine and dried ($MgSO_4$). Removal of the solvent gave a 14.7 g residue which was chroma-tographed on silica gel (220 g). Elution of the column with methylene chloride-ethyl acetate (90:10; v/v), fractions 7-15 after removal of the solvent afforded 9.8 g (80%) of racemic-2-methyl-1-[4-(phenyl-methoxy)phenyl]-4-[4-(phenylmethyl)-1-piperidinyl]-1,4-butanedione. Analytical sample was crystallized from ether, m.p. 99-100°C.

EXAMPLE 4

(R*,S*)-rac.-4-Hydroxy-4-(4-hydroxyphenyl)-3-methyl-1-[4-(phenylmethyl)-1-piperidinyl]-1-butanone

A solution of 4.0 g (0.0087 mol) of racemic-2-methyl-1-[4-(phenylmethoxy)phenyl]-4-[4-(phenylmethyl)-1-piperidinyl]-1,4-butanedione in 125 ml acetic acid was hydrogenated with 1.5 g of palladium on carbon (10%) for 5.5 hrs at 50 psi at room temperature. The catalyst was filtered and the filtrate was concentrated at reduced pressure. The residue was partitioned between methylene chloride and dilute ammonium hydroxide. The organic extracts were washed with brine and dried ($MgSO_4$). Removal of the solvent gave a 3.1 g residue which was chromatographed on silica gel (110 g). The column was eluted with methylene chloride-ethyl acetate (2:8; v/v), fractions 23-34 after removal of the solvent gave 2.3 g of crude product which was crystallized from ethyl acetate-hexane to give 2.1 g (72%) of (R*,S*)-rac.-4-hydroxy-4-(4-hydroxyphenyl)-3-methyl-1-[4-(phenylmethyl)-1-piperidinyl]-1-butanone, m.p. 128-130°C.

EXAMPLE 5

(R*,S*)-rac.-α-(4-Hydroxyphenyl)-β-methyl-4-(phenylmethyl)-1-piperidinebutanol

To a mixture of 0.7 g (0.018 mol) of lithium aluminum hydride in 10 ml of tetrahydrofuran was added dropwise a solution of 1.0 g (0.0027 mol) of (R*,S*)-rac.-4-hydroxy-4-(4-hydroxyphenyl)-3-methyl-1-[4-(phenylmethyl)-1-piperidinyl]-1-butanone in 25 ml of dry tetrahydrofuran over a period of 10 minutes. The mixture was stirred at reflux for 2 hrs then cooled to room temperature and decomposed by a dropwise addition of ethyl acetate followed by hydrochloric acid. The reaction mixture was partitioned between ethyl acetate and dilute ammonium hydroxide. The combined organic extracts were washed with brine, dried ($MgSO_4$) and the solvent was removed under reduced pressure to give 0.9 g of (R*,S*)-rac.-α-(4-hydroxyphenyl)-β-methyl-4-(phenylmethyl)-1-piperidinebutanol, which was crystallized from ethyl acetate to yield 0.6 g (63%) of pure base, m.p. 174-175°C.

0.6 g (0.0017 mol) of the above base was treated with fumaric acid to give 0.7 g (86%) of (R*,S*)-rac.-α-(4-hydroxyphenyl)-β-methyl-4-(phenylmethyl)-1-piperidinebutanol•(E)-2-butenedioate as the hemihydrate, m.p. 90-92°C.

EXAMPLE 6

rac.-4-[2-Methyl-4-[4-(phenylmethyl)-1-piperidinyl]butyl]phenol.

To a solution of 1.0 g (0.0027 mol) of (R*,S*)-rac.-4-hydroxy-4-(4-hydroxyphenyl)-3-methyl-1-[4-(phenylmethyl)-1-piperidinyl]-1-butanonein 30 ml of dry tetrahydrofuran was added dropwise over 10 minutes 3.3 ml (0.043 mol) of borane-methyl sulfide complex. The reaction mixture was stirred at room temperature for 0.5 hr then at reflux for 11 hrs. It was cooled to room temperature and decomposed by a dropwise addition of 20 ml of methanol followed by 2 ml of concentrated hydrochloric acid, which was then heated at reflux for 2 hrs. After removal of the solvent, the residue was partitioned between methylene chloride and dilute ammonium hydroxide. The combined methylene chloride extracts were washed with brine, dried ($MgSO_4$) and the solvent was removed to give 1.1 g of a residue, which was chromatographed on silica gel (35 g). Elution of the column with ethyl acetate-methylene chloride (8:2, v/v) fractions 9-22, after removal of the solvent gave the crude product, which was crystallized from ether to provide 0.75 g (83%) of rac.-4-[2-methyl-4-[4-(phenylmethyl)-1-piperidinyl]butyl]phenol, m.p. 142-143°C.

0.4 g (0.001 mol) of the above base in acetone on treatment with maleic acid afforded 0.3 g (67%) of rac.-4-[2-methyl-4-[4-(phenyl-methyl)-1-piperidinyl]butyl]phenol•maleate, m.p. 142-143°C.

EXAMPLE 7

(R*,S*)-rac.-β-Methyl-α-[4-(phenylmethoxy)phenyl]-4-(phenylmethyl)-1-piperidinepropanol

To a suspension of 0.5 g (0.013 mol) of lithium aluminum hydride in 15 ml of anhydrous tetrahydrofuran was added dropwise a solution of 1.0 g (0.0023 mol) of rac.-2-[methyl-1-[4-(phenylmethoxy)phenyl-3- [4-(phenylmethyl)-1-piperidinyl]-1-propanone in 20 ml of anhydrous tetrahydrofuran over a period of 10 minutes. The reaction mixture was stirred at room temperature for 1 hr, then it was decomposed by dropwise addition of 10 ml of ethyl acetate followed by 30 ml of brine. The mixture was extracted with ethyl acetate. The ethyl acetate solution was washed with brine, dried ($MgSO_4$) and the solvent was removed under reduced pressure to give 0.9 g a mixture of the racemic erythro and threo diastereomers in a ratio of 6:7. Chroma-tographic separation of the diastereomers was carried out on silica gel (60 g). Elution of the column with the chloroformmethanol-water-acetic acid [90:15:10:6; v/v], fractions 6 and 7 after removal of the solvent and crystallization from ether-hexane gave 0.34 g (35%) of (R*,S*)-rac.-β-methyl-α-[4-(phenylmethoxy)phenyl]-4-(phenylmethyl)-1-piperidine-propanol, m.p. 83-84°C.

A sample of the above base, on treatment with hydrogen chloride (anhydrous) in ethanol yielded (R*,S*)-rac.-β-methyl-α-[4-(phenylmethoxy)phenyl]-4-(phenylmethyl)-1-piperidinepropanol•HCl, m.p. 189-190°C.

EXAMPLE 8

(R*,S*)-rac.-α-(4-Hydroxyphenyl)-β-methyl-4-(phenylmethyl)-1-piperidinepropanol

A mixture of 0.8 g (0.0019 mol) of (R*,S*)-rac.-β-methyl-α-[4-(phenylmethoxy)phenyl]-4-(phenylmethyl)-1-piperidinepropanol, 0.8 g of ammonium formate and 0.8 g of palladium on carbon (10%) in 50 ml of acetone was stirred and refluxed for 1 hr. The catalyst was separated by filtration and the filtrate was concentrated under reduced pressure. The residue was partitioned between methylene chloride and dilute ammonium hydroxide. The methylene chloride extracts were washed with brine, dried ($MgSO_4$) then the solvent was removed under reduced pressure and the residue (0.6 g) in acetone was treated with hydrogen chloride (anhydrous). The resulting crystals were collected by filtration, dried to give 0.4 g (56%) of (R*,S*)-rac.-α-(4-hydroxy-phenyl)-β-methyl-4-(phenylmethyl)-1-piperidinepropanol•HCl, m.p. 190-191°C.

EXAMPLE 9

(R*,R*)-rac.-α-(4-Hydroxyphenyl)-β-methyl-4-(phenylmethyl)-1-piperidinepropanol.

A mixture of 0.8 g (0.019 mol) of (R*,R*)-rac.-β-methyl-α-[4-(phenylmethoxy)phenyl]-4-(phenylmethyl)-1-piperidinepropanol, 0.8 g (0.012 mol) of ammonium formate and 0.8 g of palladium on carbon (10%) in 50 ml of acetone was stirred and refluxed for 1 hr. The catalyst was separated by filtration and the filtrate was concentrated *in vacuo.* The residue was partitioned between methylene chloride and dilute ammonium hydroxide. The organic extracts were washed with brine, dried ($MgSO_4$) and the solvent was removed under reduced pressure. The crude product 0.4 g was

chromatographed on silica gel (28 g). Elution of the column with chloroform-methanol-water-acetone [90:15:10:6 (v/v)], fractions 15-30, after removal of the solvent and crystallization of the residue from ether-hexane yielded 0.15 g (24%) of (R*,R*)-rac.-α-(4-hydroxyphenyl)-β-methyl-4-(phenyl-methyl)-1-piperidinepropanol, m.p. 132-133°C.

0.5 g (0.0015 mol) of the above base and 0.17 g (0.0015 mol) of fumaric acid in ethanol afforded of (R*,R*)-rac.-α-(4-hydroxyphenyl)-β-methyl-4-(phenylmethyl)-1-piperidinepropanol•fumarate, m.p. 118-119°C.

EXAMPLE 10

rac.-4-[2-Methyl-3-[4-(phenylmethyl)-1-piperidinyl]propyl]phenol

To a solution of 2.0 g (0.059 mol) of (R*,S*)-rac.-α-(4-hydroxy-phenyl)-β-methyl-4-(phenylmethyl)-1-piperidinepropanol, in 60 ml of anhydrous tetrahydrofuran was added dropwise 6.6 ml (0.086 mol) of borane-methyl sulfide complex. The reaction mixture was stirred at room temperature for 0.5 hr then heated at reflux for 17 hrs and decomposed by dropwise addition 20 ml of methanol followed by 8.0 ml of concentrated hydrochloric acid. After refluxing the mixture for 2 hrs, it was concentrated *in vacuo* and the residue was partitioned between methylene chloride and dilute ammonium hydroxide. The organic extracts were washed with brine, dried ($MgSO_4$) and the solvent was removed under reduced pressure to give 2.0 g of crude product which was crystallized from ether to provide 1.7 g (89%) of rac.-4-[2-methyl-3-[4-(phenylmethyl)-1-piperidinyl]propyl]phenol, m.p. 119-120°C.

A sample of the above base, in acetone on treatment with hydrogen bromide (65%) gave rac.-4-[2-methyl-3-[4-(phenylmethyl)-1-piperidinyl]propyl]phenol•HBr as the hemihydrate, m.p. 118-120°C.

EXAMPLE 11

Resolution of rac.-2-methyl-1-[4-(phenylmethoxy)phenyl]-3-[4-(phenylmethyl)-1-piperidinyl]-1-propanone

A mixture of 46.0 g (0.11 mol) of rac.-2-methyl-1-[4-(phenylmethoxy)phenyl]-3-[4-(phenylmethyl)-1-piperidinyl]-1-propanone and 16.3 g (0.11 mol) of d-(+)-mandelic acid in 280 ml acetone was heated on the steam bath until clear solution obtained, then seeded with a few crystals of (+)-base d-(+)-mandelate and allowed to crystallize at room temperature for 18 hrs. The crystals were separated by filtration, washed with cold acetone and dried to yield 25.1 g (81%) of (S)-2-methyl-1-[4-(phenylmethoxy)phenyl]-3-[4-(phenylmethyl)-1-piperidinyl]-1-propanone (1:1) molar (S)-α-hydroxybenzeneacetate, m.p. 135-137°C, $[\alpha]_D^{25}$ + 42.1° (C 1.00 methanol),

EXAMPLE 12

(S)-2-Methyl-1-[4-(phenylmethoxy)phenyl]-3-[4-(phenylmethyl)-1-piperidinyl]-1-propanone

(S)-2-Methyl-1-[4-(phenylmethoxy)phenyl]-3-[4-(phenylmethyl)-1-piperidinyl]-1-propanone (S)-α-hydroxybenzeneacetate, 25.1 g (0.043 mol) was suspended in water (80 ml) and decomposed with dilute ammonium hydroxide. The resulting suspension was extracted with methylene chloride (3x200 ml). The combined methylene chloride solutions were washed with water, dried ($MgSO_4$) and removal of the solvent gave 17.4 g (94%) of (S)-2-methyl-1-[4-(phenylmethoxy) phenyl]-3-[4-(phenylmethyl-1-piperidinyl]-1-propanone, b.p. 225-230°C (0.05 mmHg), $[\alpha]_D^{25}$ + 10.9° (C 1.00 methanol).

0.7 g (0.0016 mol) of the above base in ethyl acetate was treated with hydrogen chloride (anhydrous) to give 0.72 g (95%) of (S)-2-methyl-1-[4-(phenylmethoxy)phenyl]-3-[4-(phenylmethyl)-1-piperidinyl]-1-propanone•HCl, m.p. 198-200°C, $[\alpha]_D^{25}$ + 22.9° (C 1.00 methanol).

EXAMPLE 13

(R)-2-Methyl-1-[4-(phenylmethoxy)phenyl]-3-[4-(phenylmethyl)-1-piperidinyl]-1-propanone (1:1) molar (R)-α-Hydroxybenzeneacetate

The mother liquors obtained after removal of (S)-2-methyl-1-[4-(phenylmethoxy)phenyl]-3-[4-(phenylmethyl)-1-piperidinyl]-1-propanone•(S)-α-hydroxybenzeneacetate were taken to dryness and in water (200 ml) was decomposed with dilute ammonium hydroxide. The resulting suspension was extracted with methylene chloride (2x400 ml). The combined methylene chloride solutions were washed with water, dried ($MgSO_4$) and removal of the solvent gave 26.8 g of crude (R)-2-methyl-1-[4-(phenylmethoxy)phenyl]-3-[4-phenylmethyl)-1-piperi-dinyl]-1-propanone. 26.8 g (0.062 mol) of the crude base and 9.49 g (0.062 mol) of ℓ-(-)-mandelic acid in 160 ml of acetone was heated on the steam bath until a clear solution was obtained, then seeded with a few crystals of (R)-2-methyl-1-[4-(phenylmethoxy)

phenyl]-3-[4-(phenylmethyl)-1-piperidinyl]-1-propanone (1:1) molar (R)-α-hydroxy-benzeneacetate and allowed to crystallize at room temperature for 18 hrs. The crystals were separated by filtration, washed with cold acetone and dried to yield 26.3 g (84%) of (R)-2-methyl-1-[4-(phenylmethoxy)phenyl]-3-[4-(phenylmethyl)-1-piperidinyl]-1-propanone (1:1) molar (R)-α-hydroxybenzeneacetate salt, m.p. 136-138°C. The analytical sample was recrystallized from acetone, m.p. 136-138°C, $[\alpha]_D^{25}$ -41.2°(C 1.00 methanol).

EXAMPLE 14

(R)-2-Methyl-1-[4-(phenylmethoxy)phenyl]-3-[4-(phenylmethyl)-1-piperidinyl]-1-propanone

(R)-2-Methyl-1-[4-(phenylmethoxy)phenyl]-3-[4-(phenyl-methyl)-1-piperidinyl]-1-propanone (1:1) molar (R)-α-hydroxy-benzeneacetate, 26.3 g (0.045 mol) was suspended in water (100 ml) and decomposed with dilute ammonium hydroxide. The resulting suspension was extracted with methylene chloride (3x200 ml). The combined methylene chloride solutions were washed with water (100 ml), dried ($MgSO_4$) and removal of the solvent gave 18.7 g (96%) of (R)-2-methyl-1-[4-(phenylmethoxy)phenyl]-3-[4-(phenylmethyl)-1-piperidinyl]-1-propanone, b.p. 227-231°C (0.05 mm-Hg), $[\alpha]_D^{25}$ -9.1° (C 1.00 methanol).

0.7 g (0.0016 mol) of the above base in ethyl acetate was treated with hydrogen chloride (anhydrous) to give 0.71 g (93%) of (R)-2-methyl-1-[4-(phenylmethoxy)phenyl]-3-[4-(phenylmethyl)]-1-propanone monohydrochloride, m.p. 197-199°C. The analytical sample was recrystallized from ethanol, m.p. 197-199°C, $[\alpha]_D^{25}$ -22.7° (C 1.00 methanol).

EXAMPLE 15

[R-(R*S*)]-α-(4-Hydroxyphenyl)-β-methyl-4-(phenylmethyl)-1-piperidinepropanol

A solution of 5.0 g (0.013 mol) of (S)-2-methyl-1-[4-(phenylmethoxy)phenyl]-3-[4-(phenylmethyl)-1-piperidinyl]-1-propanone in 100 ml of acetic acid was hydrogenated over 1.5 g of palladium on carbon (10%) at room temperature and 50 psi for 17 hrs. The catalyst was removed by filtration, the filtrate was concentrated *in vacuo* and the residue was partitioned between ethyl acetate and dilute ammonium hydroxide. The ethyl acetate extracts were washed with brine, dried ($MgSO_4$) and the solvent was removed to give 3.3 g of diastereomeric alcohols in a ratio of 9:1. To 3.3 g (0.0097 mol) of the crude base in acetone, was added 1.2 g (0.0098 mol) of benzoic acid. The resulting crystals were collected to give 3.7 g (62%) of [R-(R*S*)]-α-(4-hydroxyphenyl)-β-methyl-4-(phenylmethyl)-1-piperidinepropanol benzoate, m.p. 171-172°C, $[\alpha]_D^{25}$ + 15.5° (C 1.00 methanol),

1.0 g (0.0021 mol) of the above salt was partitioned between ethyl acetate and dilute ammonium hydroxide. The ethyl acetate solutions were washed with brine and dried ($MgSO_4$). Removal of the solvent gave a residue, which was crystallized from ether-hexane to yield 0.7 g (91%) of [R-(R*S*)]-α-(4-hydroxyphenyl)-β-methyl-4-(phenylmethyl)-1-piperidinepropanol, m.p. 107-108°C, $[\alpha]_D^{25}$ + 32.5° (C 1.00 methanol).

To 0.17 g (0.0005 mol) of the above base in acetone was added 0.08 g (0.0005 mol) of p-chlorobenzic acid. The resulting crystals were collected to provide 0.16 g (65%) of [R-(R*S*)]-α-(4-hydroxyphenyl)-β-methyl-4-(phenylmethyl)-1-piperidinepropanol 4-chlorobenzoate. The analytical sample was recrystallized from acetonitride, m.p. 140-142°C, $[\alpha]_D^{25}$ + 14.5° (C 1.00 methanol).

EXAMPLE 16

[S-(R*S*)]-α-(4-Hydroxyphenyl)-β-methyl-4-(phenylmethyl)-1-piperidinepropanol

A solution of 5.0 g (0.013 mol) of (R)-2-methyl-1-[4-(phenylmethoxy)phenyl-3-[4-(phenylmethyl)-1-piperidinyl]-1-propanone in 100 ml of acetic acid was hydrogenated over 1.5 g of palladium on carbon (10%) at room temperature and 50 psi for 17 hrs. The catalyst was removed by filtration, the filtrate was concentrated *in vacuo* and the residue was partitioned between ethyl acetate and dilute ammonium hydroxide. The ethyl acetate extracts were washed with brine, dried ($MgSO_4$) and the solvent was removed under reduced pressure to give 3.0 g of diastereomeric alcohols in a ratio of 9:1. To 3.0 g (0.0088 mol) of crude base in acetone was added 1.0 g (0.0088 mol) of benzoic acid. The resulting crystals were collected to give 3.6 g (60%) of pure [S-(R*S*)]-α-(4-hydroxyphenyl)-β-methyl-4-(phenyl-methyl)-1-piperidinepropanol benzoate, m.p. 171-172°C, $[\alpha]_D^{25}$ - 15.2° (C 1.00 methanol).

1.0 g (0.0021 mol) of the above base was partitioned between ethyl acetate and dilute ammonium hydroxide. The ethyl acetate solution was washed with brine and dried ($MgSO_4$). Removal of the solvent and after recrystallization from ether-hexane gave 0.7 g (91%) of [S-(R*S*)]-α-(4-hydroxyphenyl)-β-methyl-4-(phenylmethyl)-1-piperidinepropanol, m.p. 107-108°C, $[\alpha]_D^{25}$ - 32.5° (C, 1.00, methanol).

EXAMPLE 17

Racemization of (R)-2-Methyl-1-[4-(phenylmethoxy)phenyl]-3-[4-(phenylmethyl)-1-piperidinyl]-1-propanone

To 0.5 g of sodium hydroxide in 8.0 ml of methanol was added 0.5 g (0.001 mol) of (R)-2-methyl-1-[4-(phenylmethoxy)phenyl]-3-[4-(phenylmethyl)-1-piperidinyl]-1-propanone [[$\alpha]_D^{25}$ - 10.5° (C, 1.00, methanol)]. The reaction mixture was heated at reflux for 17 hrs then cooled to room temperature and diluted with brine. The aqueous suspension was extracted with ethyl acetate. The ethyl acetate solution was washed with brine, dried ($MgSO_4$) and the solvent was removed under reduced pressure to give 0.4 g (80%) of crude rac.-2-[methyl-1-[4-(phenylmethoxy)phenyl]-3-[4-(phenylmethyl)-1-piperidinyl]-1-propanone [[$\alpha]_D^{25}$ - 0.25° (C, 1.00, methanol)].

0.4 g (0.001 mol) of the above base on treatment with hydrogen chloride (anhydrous) in ethyl acetate afforded 0.35 g (65%) of rac.-2-[methyl-1-[4-(phenylmethoxy)phenyl]-3-[4-(phenylmethyl)-1-piperidinyl]-1-propanone monohydrochloride m.p. 192-193°C. .

EXAMPLE 18

rac-1-(4-(Benzyloxyphenyl)-2-methyl-3-[4-(phenylmethyl)-1-piperidinyl]-1-propanone

A mixture of 9.6 g (0.04 mol) of 4-benzyloxypropiophenone, 1.2 g of paraformaldehyde, 1.75 g (0.01 mol) of 4-benzylpiperidine hydro-chloride and 1 ml of concentrated hydrochloric acid in 40 ml dimethyl-formamide was stirred and heated at 55-60°C for 17 hours. The reaction mixture was poured onto about 200 ml 1N hydrochloric acid, then precipitate was filtered, and the solids were washed with ethyl acetate (200 ml). The aqueous solution was made basic with concentrated ammonium hydroxide and extracted with ethyl acetate. The combined ethyl acetate solutions were washed with brine, then dried ($MgSO_4$) and removal of the solvent gave the crude base, which was chroma-tographed on silica gel (70 g). Elution with ethyl acetate, fractions 8-17 afforded, after removal of the solvent, 6.0 g (60%) of rac.-1-(4-benzyloxyphenyl)-2-methyl-3-[4-(phenylmethyl)-1-piperidinyl]-1-propanone, b.p. 220-222°C (0.005 Hgmm).

6.0 g of rac.-1-(4-benzyloxyphenyl)-2-methyl-3-[4-(phenyl-methyl)-1-piperidinyl]-1-propanone (0.014 mol) in acetone, on treatment with hydrogen chloride (anhydrous) afforded 6.0 g (92%) of rac.-1-(4-benzyloxyphenyl)-2-methyl-3-[4-(phenylmethyl)-1-piperidinyl]-1-propanone•HCl, m.p. 197-198°C.

EXAMPLE 19

rac.-erythro- and threo-α-(4-Hydroxyphenyl)-β-methyl-4-(phenyl-methyl)-1-piperidinepropanol

A mixture of 1.0 g (0.0025 mol) of rac.-1-(4-benzyloxyphenyl)-2-methyl-3-[4-(phenyl-methyl)-1-piperidinyl]-1-propanone•HC1 in 100 ml of ethanol was hydrogenated over 0.4 g of palladium on carbon (10%) at room temperature and 50 psi for 17 hours. The catalyst was removed by filtration and the filtrate was concentrated *in vacuo* to give 0.7 g of a mixture of diastereomeric salts rac.-erythro-α-(4-hydroxy-phenyl)-β-methyl-4-(phenylmethyl)-1-piperidinepropanol •HC1 and rac.-threo-α-(4-hydroxyphenyl)-β-methyl-4-(phenylmethyl)-1-piperidinepropanol•HC1, which afforded, after crystallization from acetone-methanol, 0.5 g (61%) or pure rac.-erythro-α-(4-hydroxy-phenyl)-β-methyl-4-(phenylmethyl)-1-piperidinepropanol hydro-chloride as the hydrate, m.p. 185-186°C.

A crude mixture of the diastereomeric salts, rac.-erythro-α-(4-hydroxyphenyl)-β-methyl-4-(phenylmethyl)-1-piperidine-propanol •HC1 and rac.-threo-α-(4-hydroxyphenyl)-β-methyl-4-(phenylmethyl)-1-piperidinepropanol•HC1, 0.5 g (0.0013 mol), obtained from a separate run was partitioned between methylene chloride and dilute ammonium hydroxide. The methylene chloride solution was washed with brine, dried ($MgSO_4$) and the solvent was removed under reduced pressure to give 0.3 g (67%) of a mixture of diastereomeric alcohols, rac.-erythro-α-(4-hydroxyphenyl)-β-methyl-4-(phenyl-methyl)-1-piperidinepropanol and rac.-threo-α-(4-hydroxy-phenyl)-β-methyl-4-(phenylmethyl)-1-piperidinepropanol in a ratio of 5:1, which was chromatographed on silica gel (12 g). Elution with chloroform-methanol-water (90:15:10, v/v), fractions 6-14 afforded, after removal of the solvent and recrystallization of the residue from ether-hexane, 0.2 g (60%) of pure rac.-erythro-α-(4-hydroxyphenyl)-β-methyl-4-(phenylmethyl)-1-piperidinepropanol, m.p. 150-151°C.

EXAMPLE 20

rac.-1-(4-Chlorophenyl)-2-methyl-3-[4-(phenylmethyl)-1-piperidinyl]-1-propanone

A mixture of 6.7 g (0.04 mol) of 4-chloropropiophenone, 1.2 g of paraformaldehyde, 8.4 g (0.04 ml) of 4-benzylpiperidine hydrochloride in 40 ml dimethylformamide was stirred and heated in 55-60°C for 20 hours. The reaction

mixture was poured onto 1N hydrochloric acid and the precipitate was separated by filtration. The crude rac.-1-(4-chloro-phenyl)-2-methyl-3-[4-(phenylmethyl)-1-piperidinyl]-1-propanone •HC1 was partitioned between ethyl acetate and dilute ammonium hydroxide. The ethyl acetate solution was washed with brine, then dried ($MgSO_4$) and removal of the solvent gave 5.0 g (35%) of rac.-1-(4-chlorophenyl)-2-methyl-3-[4-(phenylmethyl)-1-piperidinyl]-1-propanone. For analysis a sample of this compound was distilled, b.p. 200-205°C (0.06 Hgmm).

5.0 g (0.014 mol) of rac.-1-(4-chlorophenyl)-2-methyl-3-[4-(phenylmethyl)-1-piperidinyl]-1-propanone on treatment with hydrogen chloride (anhydrous) in acetone gave 4.2 g (75%) of rac.-1-(4-chlorophenyl)-2-methyl-3-[4-(phenylmethyl)-1-piperidinyl]-1-propanone•HC1, m.p. 199-200°C. Analytical sample was recrystallized from acetone, m.p. 204-205°C.

EXAMPLE 21

rac.-1-(2-Methyl-3-phenylpropyl)-4-(phenylmethyl)piperidine

A mixture of 2.0 g (0.005 mol) of rac.-1-(4-chlorophenyl)-2-methyl-3-[4-(phenylmethyl)-1-piperidinyl]-1-propanone•HC1 in 150 ml of warm ethanol was hydrogenated (Parr Hydrogenator) over 0.8 g of palladium on carbon (10%) at room temperature and 50 psi fpr 17 hours. The catalyst was removed by filtration and the solvent concentrated to a low volume and to the mixture acetone was added. The crystals were separated by filtration, yield of rac.- 1-(2-methyl-3-phenylpropyl)-4-(phenylmethyl)piperidine•HC1 is 1.0 g (56%), m.p. 177-178°C. For analysis a sample of this compound was recrystallized from ethanol-acetone, m.p. 177-178°C.

An aliquot of the above salt rac.-1-(2-methyl-3-phenylpropyl)-4-(phenylmethyl)piperidine•HC1 was converted to the free base using ammonium hydroxide as base and ethyl acetate for extraction. For analysis, the base rac.-1-(2-methyl-3-phenylpropyl)-4-(phenyl-methyl)piperidine was distilled, b.p. 168-170°C (0.05 Hgmm).

EXAMPLE 22

Tablet Formulation (Wet Granulation)

| Item | Ingredients | mg/tablet | | | |
|---|---|---|---|---|---|
| | | 5mg | 25mg | 100mg | 500mg |
| 1. | (R*,S*)-rac-α-(4-Hydroxyphenyl)-β-methyl-4-(phenylmethyl)-1-piperidinepropanol | 5 | 25 | 100 | 500 |
| 2. | Lactose Anhydrous DTG | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 1 | 1 | 1 |
| | TOTAL | 167 | 167 | 167 | 835 |

Manufacturing Procedure:

1. Mix Items 1, 2, 3 and 4 and granulate with purified water.

2. Dry the granulation at 50°C.

3. Pass the granulation through suitable milling equipment.

4. Add Item 5 and mix for three minutes; compress on a suitable press.

EXAMPLE 23

| Capsule Formulation | | | | | |
|---|---|---|---|---|---|
| Item | Ingredients | mg/tablet | | | |
| | | 5mg | 25mg | 100mg | 500mg |
| 1. | (R*,S*)-rac-α-(4-Hydroxyphenyl)-β-methyl-4-(phenylmethyl)-1-piperidinepropanol | 5 | 25 | 100 | 500 |
| 2. | Hydrous Lactose | 159 | 123 | 148 | --- |
| 3. | Corn Starch | 25 | 35 | 40 | 70 |
| 4. | Talc | 10 | 15 | 10 | 25 |
| 5. | Magnesium Stearate | 1 | 2 | 2 | 5 |
| | TOTAL | 200 | 200 | 300 | 600 |

Manufacturing Procedure:

1. Mix Items 1, 2, and 3 in a suitable mixer for 30 minutes.

2. Add Items 4 and 5 and mix for 3 minutes.

3. Fill into a suitable capsule.

EXAMPLE 24

| Tablet Formulation (Wet Granulation) | | | | | |
|---|---|---|---|---|---|
| Item | Ingredients | mg/tablet | | | |
| | | 5mg | 25mg | 100mg | 500mg |
| 1. | [S-(R*,S*)]-α-(4-Hydroxyphenyl)-β-methyl-4-(phenylmethyl)-1-piperidinepropanol | 5 | 25 | 100 | 500 |
| 2. | Lactose Anhydrous | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 2 | 2 | 5 |
| | TOTAL | 167 | 167 | 167 | 835 |

Manufacturing Procedure:

1. Mix Items 1, 2, 3 and 4 and granulate with purified water.

2. Dry the granulation at 50°C.

3. Pass the granulation through suitable milling equipment.

4. Add Item 5 and mix for three minutes; compress on a suitable press.

**Claims**

**1.** A compound of the formula

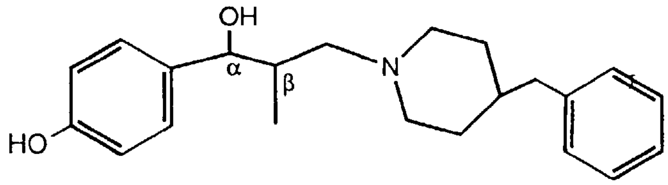

I

enantiomers, diastereomers and pharmaceutically acceptable salts thereof.

**2.** (R*,S*)-rac.-α-(4-Hydroxyphenyl)-β-methyl-4-(phenylmethyl)-1-piperidinepropanol.

**3.** [R-(R*,S*)]-α-(4-Hydroxyphenyl)-β-methyl-4-(phenylmethyl)-1-piperidinepropanol.

**4.** [S-(R*,S*)]-α-(4-Hydroxyphenyl)-β-methyl-4-(phenylmethyl)-1-piperidinepropanol.

**5.** (R*,R*)-rac-α-(4-hydroxyphenyl)-β-methyl-4-(phenylmethyl)-1-piperidinepropanol.

**6.** A compound in accordance with Claim 1 for use as therapeutically active substance.

**7.** A compound in accordance with Claim 1 for use in the treatment of neurodegenerative diseases, such as ischemia, stroke and hypoxia.

**8.** A process for the manufacture of a compound in accordance with Claim 1, which process comprises

a) reducing a compound of the formula

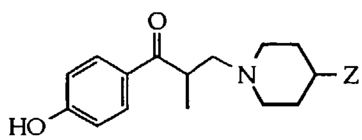

II

wherein Z is $-CH_2C_6H_5$,
with hydrogen in the presence of a suitable catalyst such as palladium on carbon, or with a complex alkali metal hydride, such as lithium aluminium or potassium borohydride, or

b) debenzylating a compound of the formula

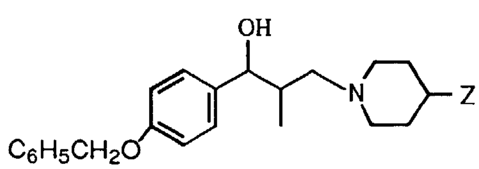

III

wherein Z is benzyl, or

c) for the manufacture of anoptically pure compound of formula I, resolving a racemic mixture into its enantiomeric components, or

d) if desired, converting a compound of formula I into a pharmaceutically acceptable salt.

**9.** A medicament containing a compound in accordance with Claim 1 and a therapeutically inert excipient.

10. A medicament reducing adverse effects of neurotoxic injury to central neurons in accordance with Claim 9 for the control or prevention of neurodegenerative diseases, such as ischemia, stroke and hypoxia.

11. The use of a compound in accordance with Claim 1 in the control or prevention of illnesses.

12. The use of a compound in accordance with Claim 1, reducing adverse effects of neurotoxic injury to central neurons in the control or prevention of neurodegenerative diseases, such as ischemia, stroke and hypoxia.

13. The use of a compound in accordance with Claim 1 for the manufacture of pharmaceutical compositions against neurodegenerative diseases, such as ischemia, stroke and hypoxia.

**Patentansprüche**

1. Eine Verbindung der Formel

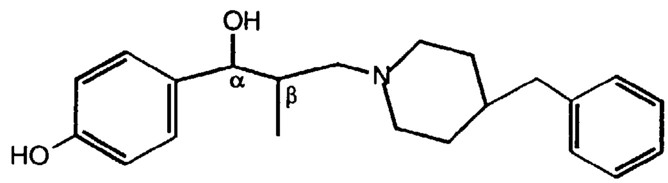 I

deren Enantiomere, Diastereomere und pharmazeutisch annehmbare Salze.

2. (R*,S*)-rac.-α-(4-Hydroxyphenyl)-β-methyl-4-(phenylmethyl)-1-piperidinpropanol.

3. [R-(R*,S*)]-α-(4-Hydroxyphenyl)-β-methyl-4-(phenylmethyl)-1-piperidinpropanol.

4. [S-(R*,S*)]-α-(4-Hydroxyphenyl)-β-methyl-4-(phenylmethyl)-1-piperidinpropanol.

5. (R*,R*)-rac-α-(4-hydroxyphenyl)-β-methyl-4-(phenylmethyl)-1-piperidinpropanol.

6. Eine Verbindung entsprechend Anspruch 1 zur Verwendung als therapeutisch aktive Substanz.

7. Eine Verbindung entsprechend Anspruch 1 zur Verwendung bei der Behandlung neurodegenerativer Erkrankungen, beispielsweise von Ischämie, Schlaganfall und Hypoxie.

8. Ein Verfahren zur Herstellung einer Verbindung entsprechend Anspruch 1, worin

a) eine Verbindung der Formel

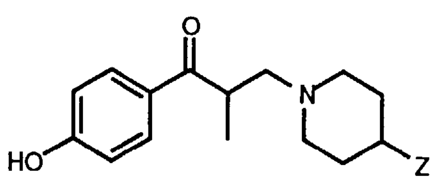 II

worin Z $-CH_2C_6H_5$ bedeutet, mit Wasserstoff in Gegenwart eines geeigneten Katalysators, beispielsweise mit einem Palladium/Kohlenstoff-Katalysator oder mit einem Alkalimetall-Hydrid-Komplex, beispielsweise mit Lithium-Aluminium oder mit Kaliumborhydrid, reduziert wird, oder

b) eine Verbindung der Formel

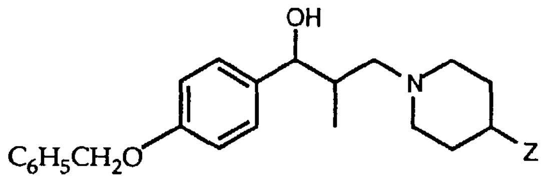

III

worin Z Benzyl bedeutet, debenzyliert wird, oder

c) optisch reine Verbindungen der Formel I erhalten werden, wenn ein racemisches Gemisch in seine enantiomeren Komponenten zerlegt wird, oder

d) erwünschtenfalls eine Verbindung der Formel I in pharmazeutisch annehmbare Salze überführt wird.

9. Ein Medikament, enthaltend eine Verbindung entsprechend Anspruch 1 und ein therapeutisches Excipient.

10. Ein Medikament zur Verringerung nachteiliger Effekte von neurotoxischen Schäden von zentralen Neuronen entsprechend Anspruch 1 für die Kontrolle oder Vorbeugung von neurodegenerativen Erkrankungen, beispielsweise Ischämie, Schlaganfall und Hypoxie.

11. Die Verwendung einer Verbindung entsprechend Anspruch 1 zur Kontrolle oder Vorbeugung von Krankheiten.

12. Die Verwendung einer Verbindung entsprechend Anspruch 1 zur Verringerung nachteiliger Effekte von neurotoxischen Schäden von zentralen Neuronen für die Kontrolle oder Vorbeugung von neurodegenerativen Erkrankungen, beispielsweise Ischämie, Schlaganfall und Hypoxie.

13. Die Verwendung einer Verbindung entsprechend Anspruch 1 zur Herstellung einer pharmazeutischen Zusammensetzung gegen neurodegenerative Erkrankungen, beispielsweise Ischämie, Schlaganfall und Hypoxie.

**Revendications**

1. Composé de formule

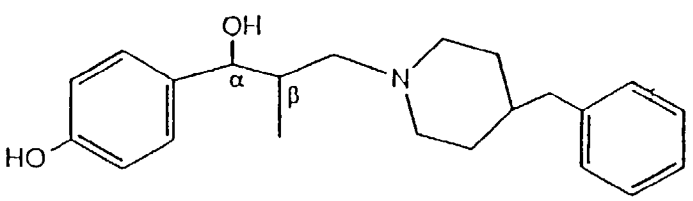

I

énantiomères, diastéréoisomères et sels pharmaceutiquement acceptables de celui-ci.

2. (R*,S*)-rac.-α-(4-hydroxyphényl)-β-méthyl-4-(phénylméthyl)-1-pipéridinepropanol.

3. [R-(R*,S*)]-α-(4-hydroxyphényl)-β-méthyl-4-(phénylméthyl)-1-pipéridinepropanol.

4. [S-(R*,S*)]-α-(4-hydroxyphényl)-β-méthyl-4-(phénylméthyl)-1-pipéridinepropanol.

5. (R*,R*)-rac.-α-(4-hydroxyphényl)-β-méthyl-4-(phénylméthyl)-1-pipéridinepropanol.

6. Composé selon la revendication 1, pour utilisation en tant que substance thérapeutiquement active.

7. Composé selon la revendication 1, pour utilisation dans le traitement de maladies neurodégénératives, telles que l'ischémie, l'attaque et l'hypoxie.

**8.** Procédé pour la préparation d'un composé selon la revendication 1, lequel procédé comprend

a) la réduction d'un composé de formule

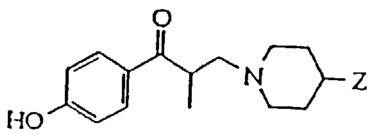

II

dans laquelle Z est $-CH_2C_6H_5$,
avec de l'hydrogène en présence d'un catalyseur approprié tel que le palladium sur charbon, ou avec un hydrure de métal alcalin complexe, tel que le borohydrure de lithium et d'aluminium ou de potassium, ou
b) la débenzylation d'un composé de formule

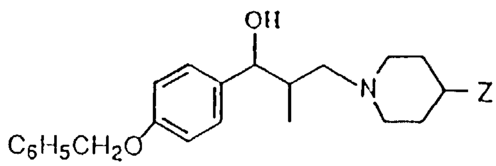

III

dans laquelle Z est le groupe benzyle, ou
c) pour la préparation d'un composé de formule I optiquement pur, la résolution d'un mélange racémique en ses composants énantiomères, ou
d) si on le désire, la conversion d'un composé de formule I en un sel pharmaceutiquement acceptable.

**9.** Médicament contenant un composé selon la revendication 1 et un excipient thérapeutiquement inerte.

**10.** Médicament réduisant les effets nuisibles d'une lésion neutotoxique de neurones centraux, selon la revendication 9, pour la lutte contre des ou la prévention de maladies neurodégénératives, telles que l'ischémie, l'attaque et l'hypoxie.

**11.** Utilisation d'un composé selon la revendication 1, dans la lutte contre des ou la prévention de maladies.

**12.** Utilisation d'un composé selon la revendication 1, réduisant les effets nuisibles d'une lésion neurotoxique des neurones centraux, dans la lutte contre des ou la prévention de maladies neurodégénératives, telles que l'ischémie, l'attaque et l'hypoxie.

**13.** Utilisation d'un composé selon la revendication 1, pour la préparation de compositions pharmaceutiques contre des maladies neurodégénératives telles que l'ischémie, l'attaque et l'hypoxie.